# EUROPÄISCHE PATENTSCHRIFT

(11) **EP 1 755 505 B1**
(45) Veröffentlichungstag und Bekanntmachung des Hinweises auf die Patenterteilung: **24.12.2008**
(21) Anmeldenummer: 05745139.5
(22) Anmeldetag: 18.05.2005
(51) Int. Cl.: A61F 5/058

(54) **HANDGELENKORTHESE**
WRIST ORTHOSIS
ORTHESE DE POIGNET

(30) Priorität: 18.06.2004 DE 102004029457
(43) Veröffentlichungstag der Anmeldung: 28.02.2007
(73) Patentinhaber: OPED AG, 6312 Steinhausen (CH)
(72) Erfinder: HASSLER, Andreas, 83101 Rohrdorf (DE); HOPMANN, Gero, 85579 Neubiberg (DE); BOTSCH, Julian, 83308 Trostberg (DE)
(74) Vertreter: Tappe, Hartmut
(86) Internationale Anmeldenummer: PCT/EP2005/005382
(87) Internationale Veröffentlichungsnummer: WO 2005/122974

(56) Entgegenhaltungen:
- WO-A-92/04880
- WO-A-03/055422
- WO-A-03/082161
- DE-A1- 4 423 755
- GB-A- 2 184 659
- US-A- 5 286 249

## Beschreibung

Die vorliegende Erfindung betrifft eine Handgelenkorthese mit einer radialwärts offenen Stützschale zur Aufnahme des distalen Unterarmbereichs und einer volaren Handstütze zur Anlage gegen die Handfläche sowie mit einer Verschlusseinrichtung zur kraftschlüssigen Verbindung der Handgelenkorthese mit dem Handgelenkbereich.

Handgelenkorthesen der eingangs genannten Art werden zur Ruhigstellung des Handgelenks eingesetzt, die beispielsweise nach handgelenksnahen Radiusfrakturen notwendig wird. Die Ruhigstellung des Handgelenkbereichs, die eine Miteinbeziehung der angrenzenden Bereiche, also des distalen Unterarmbereichs und des Mittelhandbereichs, erforderlich macht, erweist sich insbesondere betreffend den Mittelhandbereich als problematisch, da es neben einer tatsächlichen Ruhigstellung des Mittelhandbereichs auch gewünscht ist, eine Beweglichkeit der Fingergliedmaßen zu erhalten. Hinsichtlich dieser komplexen Anforderung erweist sich insbesondere die Gestaltung der Orthese in diesem Übergangsbereich als schwierig, da die Realisierung der Beweglichkeit der Fingergliedmaßen nicht einhergehen darf mit einer unzureichenden Ruhigstellung des Mittelhandbereichs am distalen Rand der Orthese.

Der nächstliegende Stand der Technik wird z.B. im WO 03/055422 beschrieben.

Der vorliegenden Erfindung liegt daher die Aufgabe zugrunde, eine Handgelenkorthese vorzuschlagen, die insbesondere im Übergangsbereich zwischen dem Handgelenk und dem Mittelhandbereich eine komfortable, die Beweglichkeit der Fingergliedmaßen ermöglichende und dennoch definierte Ruhigstellung des Mittelhandbereichs gegenüber dem Handgelenk ermöglicht.

Diese Aufgabe wird durch eine Handgelenkorthese mit den Merkmalen des Anspruchs 1 gelöst.

Die erfindungsgemäße Handgelenkorthese weist zur Auskleidung der Stützschale eine sich an den Unterarmbereich anschmiegend ausgebildete, den Handgelenkbereich umhüllende formstabile Stützeinlage auf, die sich im Handgelenkbereich ausgehend von einem Unterarmteil mit einem den Mittelhandbereich überdeckenden Mittelhandteil über die Stützschale hinaus erstreckt.

Die Ausbildung der Handgelenkorthese im Mittelhandbereich als eine sich an den Unterarmbereich anschmiegend ausgebildete, den Handgelenkbereich umhüllende formstabile Stützeinlage ermöglicht eine für eine Ruhigstellung ausreichend steife Ausgestaltung. Dabei kann die für die Stützwirkung erforderliche Formstabilität über formelastische Rückstellkräfte der Stützeinlage oder eine formsteife Ausgestaltung der Stützeinlage definiert sein, da aufgrund der abstützenden Wirkung der volaren Handstütze eine vollkommene Formsteifigkeit der Stützeinlage nicht in jedem Fall erforderlich ist. Der zur Ruhigstellung des Handgelenksbereichs notwendige radiale Druck auf die Auskleidung wird durch die Stützschale erzeugt.

Die durch die Stützeinlage erreichte angeschmiegte und somit besonders eng anliegende Abstützung sorgt insbesondere im Auslauf oder im Randbereich der Orthese dafür, dass die Realisierung der Beweglichkeit der Fingergliedmaßen nicht einhergeht mit einem Spiel zwischen Orthese und dem Mittelhandbereich.

Besonders vorteilhaft ist es, wenn der Mittelhandteil der Stützeinlage mit einer Einrichtung zur fixierenden Ausrichtung der Formkisseneinlage am Daumen versehen ist, so dass die fixierende Ausrichtung über den Daumen als Inertialpunkt für die Ausrichtung der Handgelenkorthese nutzbar ist.

Bei einer möglichen Ausführungsform der Handgelenkorthese ist die Stützeinlage aus einem elastischen Kunststoffmaterial gebildet. Je nach Art und Position der Fraktur im Handgelenkbereich kann sich die mit dem Kunststoffmaterial erzeugte abstützende Wirkung als ausreichend erweisen.

Bei einer anderen möglichen Ausführungsform der Handgelenkorthese ist die Stützeinlage aus einer evakuierbaren Formkisseneinlage mit einer Formkörperfüllung aus einer Vielzahl von Formkörpern gebildet. Hierdurch wird eine extrem formsteife und dennoch eng angeschmiegte Ausbildung der Stützeinlage möglich.

Eine Ausgestaltung der Stützschale, die einerseits eine wirksame volare und dorsale Druckbeaufschlagung des distalen Unterarmbereichs bei gleichzeitig hohem Anlegekomfort ermöglicht, besteht vorteilhafterweise darin, die Stützschale ulnarwärts mit einem durchgehend ausgebildeten Stützbereich zu versehen, von dem dorsalwärts und volarwärts zumindest eine Lasche abgeht.

Als besonders vorteilhaft sowohl für eine exakte volare Positionierung als auch eine exakte Einstellung der Handflexion erweist es sich, wenn die Stützschale am distalen Ende mit einem in seiner Relativanordnung verstellbaren Ausleger zur Anordnung der volaren Handstütze versehen ist.

Wenn darüber hinaus der Ausleger verschwenkbar an der Stützschale angeordnet ist mit einer im Wesentlichen koaxial zur Achse der Dorsalflexion verlaufenden Schwenkachse, ermöglicht der Ausleger eine der Anatomie angepasste Einstellung der Dorsalflexion.

Je nach Art der zu therapierenden Verletzung im Handgelenkbereich, beispielsweise insbesondere bei einer distalen Radiusfraktur, kann es sich als vorteilhaft erweisen, zur zirkulären Ergänzung der Stützschale eine ulnarwärts offene Komplementärschale vorzusehen, die radialwärts einen durchgehend ausgebildeten Stützbereich aufweist, von dem dorsal und volar zumindest eine Lasche abgeht, und die kraftschlüssig mit der Stützschale verbindbar ist. Durch die zirkuläre Ergänzung der Stützschale wird insbesondere der distale Radiusbereich stärker versteifend gestützt.

Eine besonders kompakte Ausgestaltung der durch die Komplementärschale zirkulär ergänzten Handgelenkorthese wird möglich, wenn zur kraftschlüssigen Verbindung der Komplementärschale mit der Stützschale ein zumindest teilweiser Übergriff zwischen zumindest einer Laschenpaarung umfassend eine dorsale und eine volare Lasche der Stützschale und einer Laschenpaarung umfassend eine dorsale und volare Lasche der Komplementärschale vorgesehen ist und zur Fixierung des Übergriffs die Verschlusseinrichtung der Stützschale dient.

Eine weitere Stabilisierung des Übergriffs kann erfolgen, wenn zur Herstellung des Übergriffs zwischen den Laschenpaarungen ein Eingriff ausgebildet ist, derart, dass die Laschen der Stützschale in eine an den Laschen der Komplementärschale ausgebildete Laschenführung eingreifen oder umgekehrt.

Eine Erhöhung der Funktionalität der Komplementärschale wird erreicht, wenn die Komplementärschale zumindest zwei Laschenpaarungen aufweist, wobei die proximale Laschenpaarung zur Herstellung der Eingriffsverbindung dient und eine dorsale und/oder volare Lasche einer distalen Laschenpaarung mit einer Andruckeinrichtung versehen ist, die eine Druckkrafteinstellung aufweist.

Hierdurch ist es möglich, insbesondere bei einer Fraktion des distalen Ulnus oder Radius im unmittelbaren Frakturbereich eine erhöhte Druckkraft einzustellen, wodurch das vom konventionellen Eingipsen des Handgelenksbereichs her bekannte "Nachdrücken" des Gipses simuliert werden kann.

Eine noch exaktere Simulation des "Nachdrückens" wird möglich, wenn die Andruckeinrichtung in ihrer Relativposition zur Lasche veränderbar ist.

Eine effektive und zugleich hinsichtlich der Ausführung einfache sowie eine hohe Betriebssicherheit gewährleistende Ausführung der Andruckeinrichtung ist radial verschiebbar mit der Lasche verbunden.

Nachfolgend wird eine bevorzugte Ausführungsform der Handgelenkorthese unter Bezugnahme auf die Zeichnung näher erläutert.

Es zeigen:
- **Fig. 1**: eine Stützschale der Handgelenkorthese mit Handstütze in volarer Ansicht;
- **Fig. 2**: die in **Fig. 1** dargestellte Stützschale in dorsaler Ansicht;
- **Fig. 3**: die in den **Fig. 1** und **2** dargestellte Stützschale in ulna- rer Ansicht;
- **Fig. 4**: eine Komplementärschale zur Kombination mit der in den **Fig. 1**, **2** und **3** dargestellten Stützschale;
- **Fig. 5**: die mit der Komplementärschale zirkulär ergänzte Stütz- schale in dorsaler Ansicht;
- **Fig. 6**: eine Handgelenkorthese aus einer Stützschale mit Form- kisseneinlage in volarer Ansicht;
- **Fig. 7**: die in **Fig. 6** dargestellte Handgelenkorthese in dorsaler Ansicht;
- **Fig. 8**: die in den **Fig. 6** und **7** dargestellte Formkisseneinlage in ebener Abwicklung.

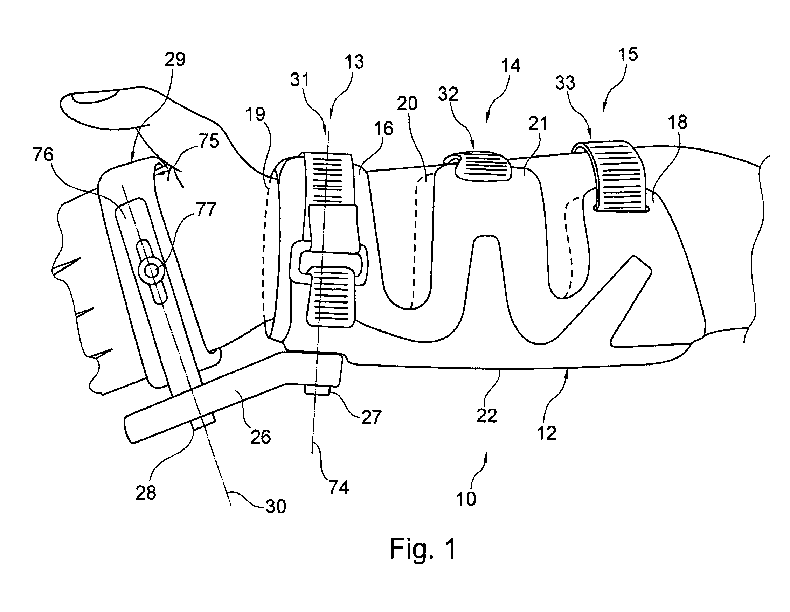

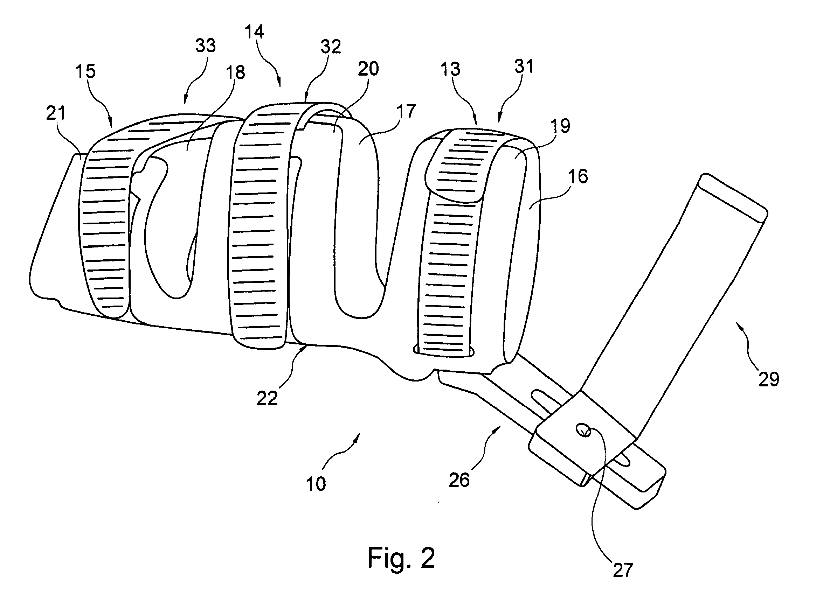

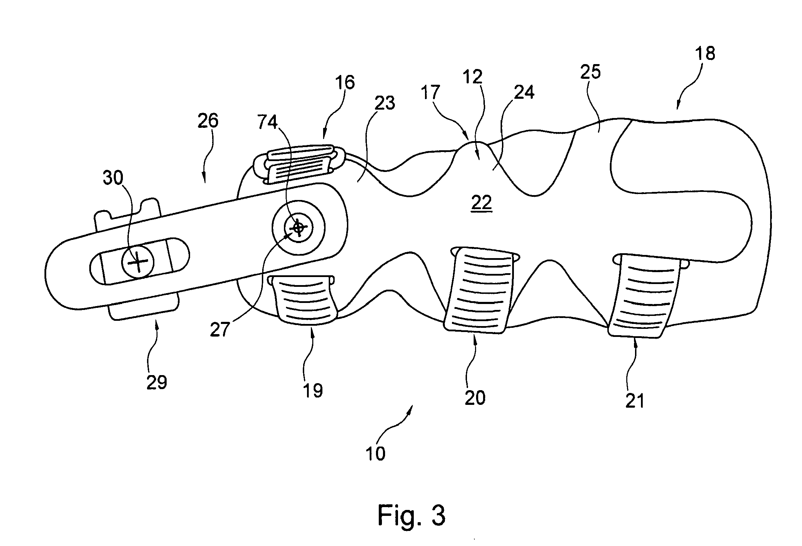

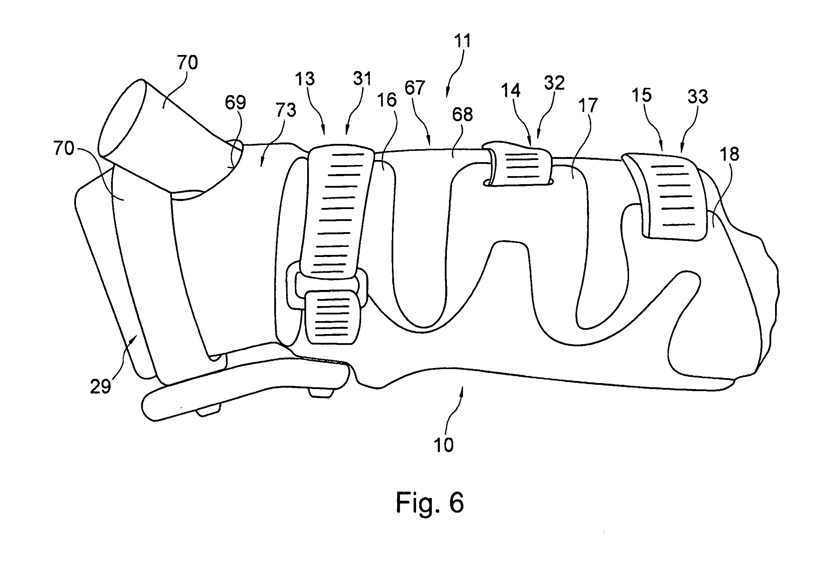

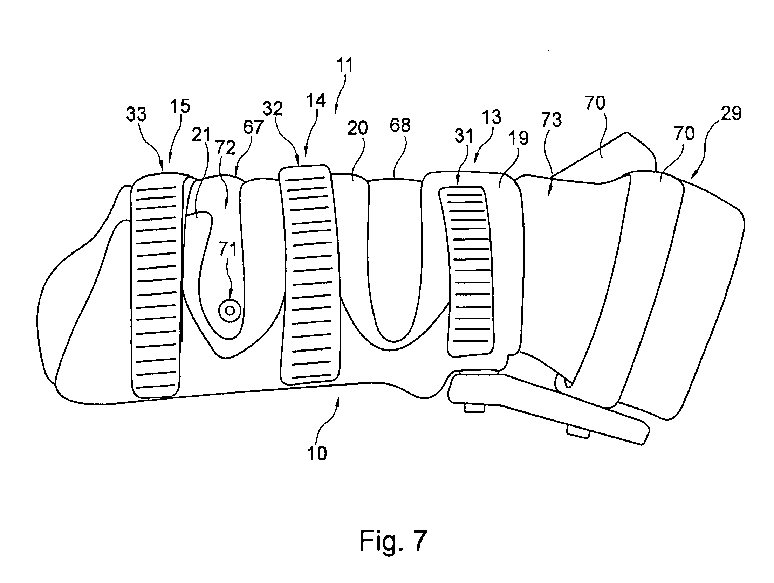

**Fig. 1** zeigt eine Stützschale 10 einer in den **Fig. 6** und **7** in ihrer Gesamtheit dargestellten Handgelenkorthese 11 in volarer Ansicht. Wie ergänzend durch die **Fig. 2** und **3** dargestellt, die die Stützschale 10 in einer dorsalen und einer ulnaren Ansicht zeigen, ist die Stützschale 10 im Wesentlichen so aufgebaut, dass, wie durch die in **Fig. 1** angedeutete Unterarmkontur deutlich wird, ausgehend von einem der Elle (Ulna) zugeordneten Stützbereich 12 insgesamt drei Laschenpaarungen 13, 14 und 15 vorgesehen sind, die jeweils eine der Unterseite des Unterarms zugeordnete volare Lasche 16, 17 und 18 sowie eine jeweils der Oberseite des Unterarms zugeordnete dorsale Lasche 19, 20, 21 aufweisen. Wie insbesondere aus der in **Fig. 3** dargestellten ulnaren Ansicht der Stützschale 10 deutlich wird, ist der Stützbereich 12 rückgratartig ausgebildet mit einem sich längs der Ulna erstreckenden Stützstrang 22 und dorsalwärts bzw. volarwärts davon ausgehenden Stützrippen 23, 24, 25, die jeweils in eine volare oder dorsale Lasche 16 bis 21 übergehen und auf diese Art und Weise versteifende Verstärkungen der betreffenden Laschen 16 bis 21 bilden.

Wie ferner aus den **Fig. 1** bis **3** zu ersehen, ist am distalen Ende des Stützbereichs 12 ein entsprechend der Handkontur von der Elle leicht nach außen abgewinkelter Ausleger 26 angeordnet, dessen proximales Ende über eine arretierbare Drehgelenkeinrichtung 27, die eine zur Achse der Dorsalflexion koaxial verlaufende Schwenkachse 74 aufweist, mit dem distalen Ende des Stützstrangs 22 verbunden ist. Wie insbesondere die **Fig. 1** und **3** zeigen, ist über eine arretierbare Gleitgelenkeinrichtung 28 eine Handstütze 29, die zur Anlage gegen die Handinnenfläche dient, so mit dem Ausleger 26 verbunden, dass sowohl eine Relativverschiebung der Handstütze 29 in Längsrichtung des Auslegers 26 als auch eine Drehung der Handstütze 29 um eine Feststellachse 30 der Gleitgelenkeinrichtung 28 möglich ist. Die Handstütze 29 weist eine Kontaktplatte 75 zur Anlage an der Handinnenfläche auf. Die Kontaktplatte 75 ist gegenüber einer Handstützenbasis 76 vermittels einer arretierbaren Gleitgelenkeinrichtung 77 verstellbar, um eine Anpassung der Handstütze 29 an die jeweilige Breite der Hand zu ermöglichen.

Wie beispielsweise aus der Darstellung gemäß **Fig. 1** deutlich wird, ist eine Installation der Stützschale 10 am Unterarm durch ein seitliches Aufschieben der Stützschale 10 bei gleichzeitigem Aufspreizen der aus einem formelastischen Kunststoffmaterial gefertigten und einstückig mit dem Stützbereich 12 verbundenen Laschenpaarungen 13 bis 15 möglich. Bei installierter Stützschale 10 liegt der Stützbereich 12 der Stützschale 10 ulnusseitig am Unterarm und die Handstütze 29 an der Handfläche an. Wie insbesondere aus **Fig. 3** ersichtlich, ermöglicht die verschwenkbare Anlenkung des Auslegers 26 an der Stützschale 10 mittels der Drehgelenkeinrichtung 27 bei Bedarf beispielsweise die Einstellung einer leichten Palmarflexion.

Wie ferner aus einer Zusammenschau der **Fig. 1**, **2** und **3** deutlich wird, sind die Laschenpaarungen 13, 14, 15 jeweils mit einer hier als Klettverschlussband ausgeführten Schließeinrichtung 31, 32, 33 versehen, die es ermöglicht, die freien Enden der Laschen 16, 19; 17, 20; 18, 21 einer Laschenpaarung 13, 14, 15 gegeneinander zu ziehen, um die Stützschale 10 in ihrer installierten Position zu sichern und je nach Zugbeaufschlagung der Schließeinrichtungen 31, 32 und 33 eine Druckbeaufschlagung des Unterarm-/Handgelenkbereichs vermittels der Laschenpaarungen 13, 14 und 15 einzustellen.

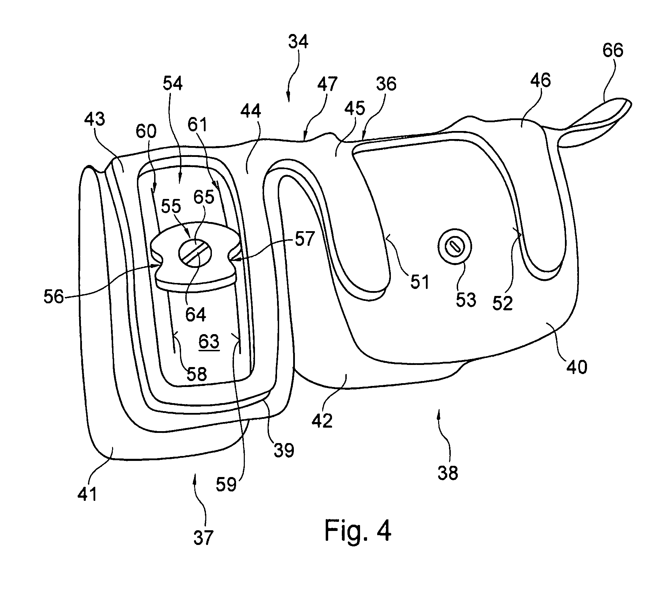

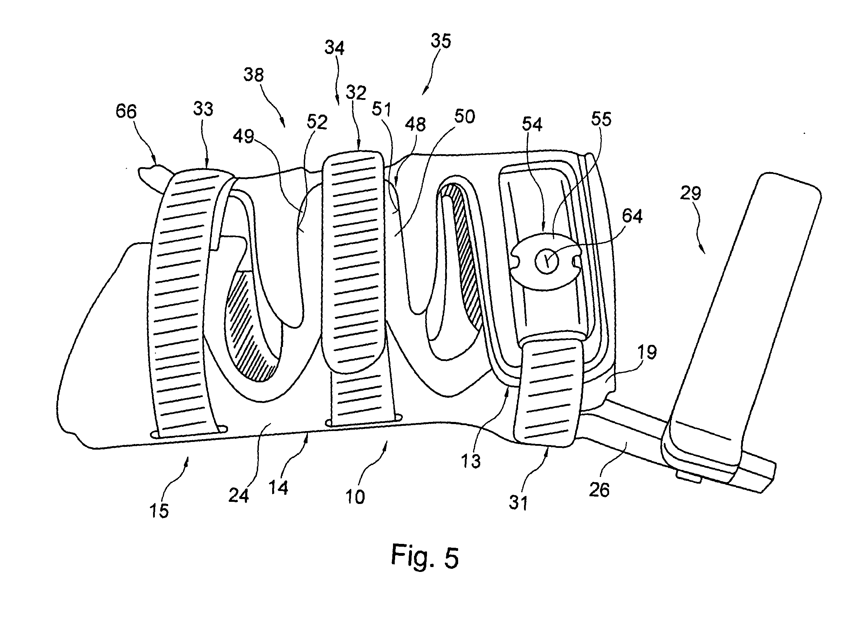

**Fig. 4** zeigt eine Komplementärschale 34 aus formelastischem Kunststoffmaterial, die, wie in **Fig. 5** dargestellt, zur zirkulären Ergänzung der Stützschale 10 dient und somit eine den Unterarm-/Handgelenkbereich allseitig umschließende Stützschalenanordnung 35 bildet. Wie **Fig. 4** zeigt, weist die Komplementärschale 34 einen Stützbereich 36 auf, der mit einer distalen und einer proximalen Laschenpaarung 37, 38 versehen ist. Sowohl die distale als auch die proximale Laschenpaarung 37, 38 umfassen jeweils eine volare Lasche 39 bzw. 40 sowie eine dorsale Lasche 41 bzw. 42. Die Laschen 39, 40, 41, 42 weisen jeweils zwei die Laschen versteifende Stützrippen 43, 44 bzw. 45, 46 auf, die von einem im Stützbereich 36 ausgebildeten Stützstrang 47 ausgehen.

Zur kraftschlüssigen Verbindung der Komplementärschale 34 mit der Stützschale 10 ist, wie in **Fig. 5** dargestellt, zwischen der proximalen Laschenpaarung der Komplementärschale 34 und der mittleren Laschenpaarung 14 der Stützschale 10 eine Eingriffsverbindung 48 ausgebildet, derart, dass die volare Lasche 17 und die dorsale Lasche 20 der mittleren Laschenpaarung 14 der Stützschale 10 mit Laschenlängsrändern 49, 50 in jeweils an den Längsrändern der volaren Lasche 40 und der dorsalen Lasche 42 der proximalen Laschenpaarung 38 der Komplementärschale 34 ausgebildete Gleitführungen 51, 52 eingreifen. Wie insbesondere **Fig. 4** zeigt, sind die Gleitführungen 51, 52 durch oberflächenparallele Einschnitte in die Stützrippen 45, 46 der proximalen Laschenpaarung 38 gebildet. Ein auf der Laschenoberfläche der volaren Lasche 40 und der dorsalen Lasche 42 ausgebildeter Rastnoppen 53, der in eine nicht näher dargestellte, durch die Ausformung der Stützrippe 24 der Laschen 20 und 17 der mittleren Laschenpaarung 14 der Stützschale 10 gebildete Vertiefung eingreift, sorgt für eine verliersichere Verbindung zwischen der Stützschale 10 und der Komplementärschale 34, ohne dass hierzu die in **Fig. 5** dargestellten Schließeinrichtungen 31, 32, 33 verwendet werden müssten.

Wie **Fig. 4** zeigt, weisen sowohl die volare Lasche 39 als auch die dorsale Lasche 41 der distalen Laschenpaarung 37 der Komplementärschale 34 eine Andruckeinrichtung 54 auf, die ein Gleitstück 55 umfasst, welches mit einem distalen und einem proximalen Endbereich 56, 57 jeweils einen Führungsrand 58, 59 eines quer zur Längserstreckung verlaufenden Laschenschlitzes 60, 61 umfasst. Dabei liegt ein in **Fig. 4** nicht dargestellter, entsprechend einem in **Fig. 4** dargestellten Oberteil 62 des Gleitstücks 55 ausgebildeter Unterteil unter Vorspannung an einer durch die Laschenschlitze 60, 61 definierten Laschenzunge 63 an. Zusammen mit der unter Vorspannung ausgebildeten Anlage des Gleitstücks 55 gegen die Laschenzunge 63 sorgt die Verbindung der Endbereiche 56, 57 des Gleitstücks 55 mit den Führungsrändern 58, 59 für eine verliersichere Verbindung zwischen dem Gleitstück 55 und den Laschen 39, 41 der distalen Laschenpaarung 37.

Zur Veränderung der Vorspannung, mit der das Gleitstück 55 gegen die Laschenzunge 63 anliegt, ist das Gleitstück 55 mit einer Druckschraube 64 versehen, die in einem hier nicht näher dargestellten Gleitstückgewinde einer Gleitstückdurchgangsbohrung 65 aufgenommen ist. Die hier nach Art einer Madenschraube ausgeführte Druckschraube 64 ermöglicht durch ein Eindrehen in die Durchgangsbohrung 65 des Gleitstücks 55 eine Erhöhung der Vorspannung zwischen dem Gleitstück 55 und der Laschenzunge 63 und somit eine relative Vorwölbung der Laschenzunge 63 in Richtung auf den Handgelenkbereich. Über eine Verschiebung des Gleitstücks 55 relativ zu den Führungsrändern 58, 59 kann der Ort dieser Vorwölbung und somit der Ort der Druckbeaufschlagung im Handgelenksbereich bestimmt werden.

**Fig. 5** zeigt, dass bei einer zirkulären Ergänzung der Stützschale 10 durch die Komplementärschale 34 die Andruckeinrichtung 54 auf die jeweils zugeordnete volare Lasche 16 und dorsale Lasche 19 der distalen Laschenpaarung 13 der Stützschale 10 wirkt. Ferner zeigt **Fig. 5**, dass bei der dargestellten Stützschalenanordnung 35 ein am proximalen Ende der Komplementärschale 34 ausgebildeter Stützstrangfortsatz 66 radial gegenüberliegend der proximalen Laschenpaarung 15 der Stützschale 10 angeordnet ist und vermittels der der proximalen Laschenpaarung 15 zugeordneten Schließeinrichtung 33 kraftschlüssig mit der Laschenpaarung 15 verbindbar ist.

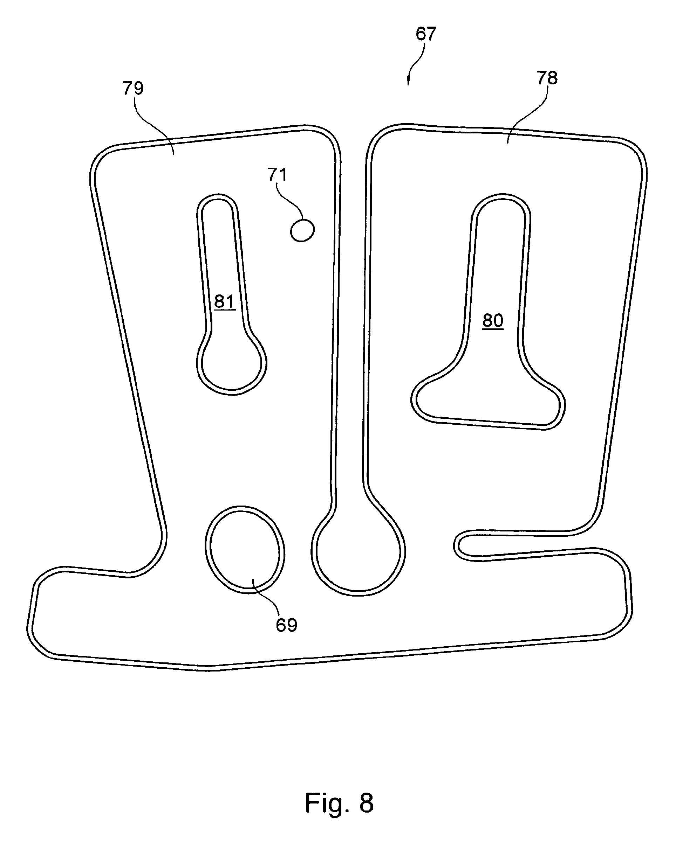

In den **Fig. 6** und **7** ist die Handgelenkorthese 11 in einer möglichen Konfiguration zur Installation am Unterarm-/Handgelenkbereich dargestellt, in der die Handgelenkorthese 11 aus der Stützschale 10 und einer zur Auskleidung der Stützschale 10 dienenden, evakuierbaren Formkisseneinlage 67 gebildet ist. Das Anlegen der in den **Fig. 6** und **7** dargestellten Handgelenksorthese 11 kann in zwei Schritten erfolgen. Im ersten Schritt wird die mit einer hier nicht näher dargestellten Formkörperfüllung aus einer Vielzahl von vorzugsweise elastischen Formkörpern versehene, in ihrer ebenen Abwicklung beispielhaft in **Fig. 8** dargestellte Formkisseneinlage 67, die zur Erhöhung des Tragekomforts mit einem textilen Überzug 68 versehen ist, am Unterarm-/Handgelenkbereich des Patienten angelegt. Dabei dient eine in der Formkisseneinlage 67 ausgebildete Aufnahmeöffnung 69 zum Durchführen des Daumens. Anschließend wird die belüftete Formkisseneinlage um den Unterarm-/Handgelenkbereich des Patienten unter Ausbildung einer Umhüllung des Unterarm-/Handgelenkbereichs herumgeschlagen und in dieser Relativanordnung durch an den Rändern der Formkisseneinlage angeordnete, hier nicht näher dargestellte Klettverschlusseinrichtungen gesichert. Dabei ist dann der Daumen durch die Aufnahme 69 der Formkisseneinlage 67 hindurchgeführt und in einer am Überzug 68 ausgebildeten Tülle 70 aufgenommen.

Zur Ergänzung der am Unterarm-/Handgelenkbereich installierten Formkisseneinlage 67 mit der Stützschale 10 wird diese von der ulnaren Seite des Unterarm-/Handgelenkbereichs her auf den Unterarm-/Handgelenkbereich aufgeschoben, und es erfolgt eine Ausrichtung bzw. Anpassung der Position der Handstütze 29 an die Patientenanatomie. Ausgehend von dieser Relativpositionierung der Stützschale 10 am Unterarm-/Handgelenkbereich vermittels der formelastischen Ausbildung der Laschenpaarungen 13 bis 15 der Stützschale 10 erfolgt eine kraftschlüssige Verbindung der jeweiligen volaren und dorsalen Laschen 16, 17, 18 bzw. 19, 20, 21 der Laschenpaarungen 13, 14, 15 vermittels der Schließeinrichtungen 31, 32 und 33 sowie eine kraftschlüssige Verbindung der Handstütze 29 mit der Hand des Patienten vermittels einer, wie in den **Fig. 6** und **7** dargestellt, an der Handstütze 29 angeordneten weiteren, hier als Klettverschlussband ausgebildeten, Schließeinrichtung 70.

Wie **Fig. 7** zeigt, weist die Formkisseneinlage 67 eine Ventileinrichtung 71 zur Evakuierung auf. Die Ventileinrichtung 71 kann so an der Formkisseneinlage 67 angeordnet sein, dass bei der mit der Formkisseneinalge 67 kombinierten Anordnung der Stützschale 10 die Ventileinrichtung 71 in einer zwischen der dorsalen Lasche 21 der proximalen Laschenpaarung 15 und der dorsalen Lasche 20 der mittleren Laschenpaarung 14 ausgebildeten Ausnehmung 72 zu liegen kommt. Durch Anschluss einer geeigneten Evakuierungseinrichtung, wie beispielsweise einer Vakuumpumpe, wird die bis dahin flexible Formkisseneinlage 67 in der in Fig. 7 dargestellten Konfiguration fixiert. Im Zusammenwirken mit der äußeren Abstützung durch die Stützschale 10 ergibt sich somit über den gesamten Bereich der Stützschale 10 und einen die Stützschale 10 distal überragenden Mittelhandbereich 73 der Formkisseneinlage 67 eine formsteife, an die Anatomie des Patienten angepasste Abstützung des Unterarm-/Handgelenkbereichs, die insbesondere über das distale Ende der Stützschale 10, also auch über den distalen Rand der distalen Laschenpaarung 13 hinaus, für eine genaue Fixierung der Relativposition des Mittelhandbereichs 73 gegenüber dem Handgelenk sorgt.

Alternativ zu der vorstehend beschriebenen Verfahrensweise kann die Formkisseneinlage 67 jedoch auch - vorteilhaft in dem textilen Überzug 68 aufgenommen - vor dem Anlegen der Stützschale 10 als Auskleidung in dieser angeordnet werden, um dann nachfolgend zusammen mit der Stützschale 10 auf den Unterarm-/Handgelenkbereich aufgeschoben zu werden.

Wie der in **Fig. 8** beispielhaft dargestellte Zuschnitt der Formkisseneinlage 67 zeigt, weist die Formkisseneinlage 67 ausgehend von dem Mittelhandteil 73, der mit der Aufnahme 69 versehen ist, einen ulnaren Einlagenteil 78 und einen radialen Einlagenteil 79 auf, die bei angelegter Formkisseneinlage 67 einander gegenüberliegend ulnar und radial am Unterarm anliegen. Darüber hinaus sind die Einlagenteile 78, 79 jeweils mit Belüftungsausnehmungen 80, 81 versehen. Die Einlagenteile 78, 79 weisen eine Relativanordnung und Dimensionierung auf, so dass es bei angelegter Formkisseneinlage 67 zu keiner volaren und dorsalen Überlappung kommt.

Je nach Ursache für die erforderliche Ruhigstellung des Handgelenkbereichs, also beispielsweise bei einer Ulnafraktur im Handgelenkbereich, kann, wie in **Fig. 5** dargestellt, eine zirkuläre Ergänzung der Stützschale 10 durch die Komplementärschale 34 ergänzt durch die in den **Fig. 6** und 7 dargestellte Auskleidung der Stützschalenanordnung durch die Formkisseneinlage erfolgen.

Bei einer von der Darstellung in den **Fig. 6** und **7** abweichenden zirkulären Konfiguration der Handgelenkorthese kann, wie etwa aus **Fig. 5** deutlich wird, vermittels einer geeigneten Einstellung bzw. Relativpositionierung der Andruckeinrichtung 54 das von der konventionellen Gipsverbandtherapie her bekannte "Nachdrücken" zur Erzielung der damit verbundenen Vorteile realisiert werden. Bei einer Kontrastmittelapplikation auf die Andruckeinrichtung 54 bzw. das Gleitstück 55 oder die Druckschraube 64 kann darüber hinaus vermittels einer Röntgenaufnahme überprüft werden, ob die durch das Andrücken erreichte Druckspitze auch exakt zum Bruch positioniert ist.

## Patentansprüche

1. Handgelenkorthese (11) mit einer radialwärts offenen Stützschale (10) zur Aufnahme des distalen Unterarmbereichs und einer volaren Handstütze (29) zur Anlage gegen die Handfläche sowie mit einer Verschlusseinrichtung (31, 32, 33, 70) zur kraftschlüssigen Verbindung der Handgelenkorthese mit dem Handgelenkbereich,
**dadurch gekennzeichnet,**
**dass** zur Auskleidung der Stützschale (10) eine sich an den Unterarmbereich anschmiegend ausgebildete, den Handgelenkbereich umhüllende formstabile Stützeinlage vorgesehen ist, die sich ausgehend von einem Unterarmteil mit einem den Mittelhandbereich überdeckenden Mittelhandteil (73) über die Stützschale (10) hinaus erstreckt.

2. Handgelenkorthese nach Anspruch 1,
**dadurch gekennzeichnet,**
**dass** der Mittelhandteil (73) der Stützeinlage mit einer Einrichtung (69) zur fixierenden Ausrichtung der Stützeinlage am Daumen versehen ist.

3. Handgelenkorthese nach Anspruch 1 oder 2,
**dadurch gekennzeichnet,**
**dass** die Stützeinlage aus einem elastischen Kunststoffmaterial gebildet ist.

4. Handgelenkorthese nach Anspruch 1 oder 2,
**dadurch gekennzeichnet,**
**dass** die Stützeinlage aus einer evakuierbaren Formkisseneinlage (67) mit einer Formkörperfüllung aus einer Vielzahl von Formkörpern gebildet ist.

5. Handgelenkorthese nach einem der vorangehenden Ansprüche,
**dadurch gekennzeichnet,**
**dass** die Stützschale (10) ulnarwärts einen durchgehend ausgebildeten Stützbereich (12) aufweist, von dem dorsalwärts und volarwärts zumindest eine Lasche (16, 17, 18, 19, 20, 21) abgeht.

6. Handgelenkorthese nach einem der vorangehenden Ansprüche,
**dadurch gekennzeichnet,**
**dass** die Stützschale (10) am distalen Ende mit einem in seiner Relativanordnung verstellbaren Ausleger (26) zur Anordnung der volaren Handstütze (29) versehen ist.

7. Handgelenkorthese nach Anspruch 5,
**dadurch gekennzeichnet,**
**dass** der Ausleger (26) verschwenkbar an der Stützschale (10) angeordnet ist mit einer im Wesentlichen koaxial zur Achse der Dorsalflexion verlaufenden Schwenkachse (74).

8. Handgelenkorthese nach einem der vorangehenden Ansprüche,
**dadurch gekennzeichnet,**
**dass** zur zirkulären Ergänzung der Stützschale (10) eine ulnarwärts offene Komplementärschale (34) vorgesehen ist, die radiuswärts einen durchgehend ausgebildeten Stützbereich (36) aufweist, von dem dorsalwärts und volarwärts zumindest eine Lasche (39, 40, 41, 42) abgeht, und die kraftschlüssig mit der Stützschale verbindbar ist.

9. Handgelenkorthese nach Anspruch 8,
**dadurch gekennzeichnet,**
**dass** zur kraftschlüssigen Verbindung der Komplementärschale (34) mit der Stützschale (10) ein zumindest teilweiser Übergriff zwischen zumindest einer Laschenpaarung (14) aus einer dorsalen und volaren Lasche (17, 20) der Stützschale und einer Laschenpaarung (37) aus einer dorsalen und volaren Lasche (40, 42) der Komplementärschale vorgesehen ist, und die Verschlusseinrichtung (32) der Stützschale zur Fixierung des Übergriffs dient.

10. Handgelenkorthese nach Anspruch 9,
**dadurch gekennzeichnet,**
**dass** zur Herstellung des Übergriffs zwischen den Laschenpaarungen (14, 37) ein Eingriff ausgebildet ist, derart, dass die Laschen (17, 20) der Stützschale (10) in eine an den Laschen (40, 42) der Komplementärschale (34) ausgebildete Laschenführung (51, 52) eingreifen oder umgekehrt.

11. Handgelenkorthese nach einem der Ansprüche 8-10,
**dadurch gekennzeichnet,**
**dass** die Komplementärschale (34) zwei Laschenpaarungen (37, 38) aufweist, wobei die dorsale und/oder volare Lasche (39, 41) der distalen Laschenpaarung (37) mit einer Andruckeinrichtung (54) versehen ist, die eine Druckkrafteinstellung (64) aufweist.

12. Handgelenkorthese nach Anspruch 11,
**dadurch gekennzeichnet,**
**dass** die Andruckeinrichtung (54) in ihrer Relativposition zur Lasche (39, 41) veränderbar ist.

13. Handgelenkorthese nach Anspruch 12,
**dadurch gekennzeichnet,**
**dass** die Andruckeinrichtung (54) radial verschiebbar mit der Lasche (39, 41) verbunden ist.

## Claims

1. A wrist orthosis (11) having a supporting shell (10) that is open in the radial direction to receive the distal forearm area and having a volar hand support (29) for coming into contact with the palmar surface of the hand and having a closing device (31, 32, 33, 70) for force-locking connection of the wrist orthosis to the wrist area,
**characterized in that**
to line the supporting shell (10), there is provided a dimensionally stable supporting insert that surrounds the wrist area and is designed to conform to the forearm area, the supporting insert extending beyond the supporting shell (10) starting from a forearm part with a metacarpal part (73) that covers the metacarpal area.

2. The wrist orthosis according to Claim 1,
**characterized in that**
the metacarpal part (73) of the supporting insert is provided with a device (69) for fixational alignment of the supporting insert on the thumb.

3. The wrist orthosis according to Claim 1 or 2,
**characterized in that**
the supporting insert is made of an elastic plastic material.

4. The wrist orthosis according to Claim 1 or 2,
**characterized in that**
the supporting insert is formed from an evacuable moulded cushion insert (67) with a moulded body filling consisting of a plurality of moulded bodies.

5. The wrist orthosis according to any one of the preceding claims,
**characterized in that**
ulnarly the supporting shell (10) has a supporting area (12) that is designed continuously, with at least one strap (16, 17, 18, 19, 20, 21) proceeding in the dorsal and volar directions therefrom.

6. The wrist orthosis according to any one of the preceding claims,
**characterized in that**
the supporting shell (10) is provided on its distal end with a cantilevered portion (26) that is adjustable in its relative arrangement for arrangement of the volar hand support (29).

7. The wrist orthosis according to Claim 5,
**characterized in that**
the cantilevered portion (26) is pivotably arranged on the supporting shell (10) with a pivot axis (74) running essentially coaxially with the axis of the dorsal flexion.

8. The wrist orthosis according to any one of the preceding claims,
**characterized in that**
for circular supplementation of the supporting shell (10), a complementary shell (34) is provided, said complementary shell being open ulnarly and having a continuous supporting area (36) radially, with at least one strap (39, 40, 41, 42) proceeding in dorsal and volar directions therefrom and said complementary shell being connectable in a force-locking manner to the supporting shell.

9. The wrist orthosis according to Claim 8,
**characterized in that**
for force-locking connection of the complementary shell (34) to the supporting shell (10), at least a partial overlap is provided between at least one pair of straps (14) comprising a volar strap (17) and a dorsal strap (20) of the supporting shell, and a pair of straps (37) comprising a dorsal and volar strap (40, 42) of the complementary shell, and the closing device (32) of the supporting shell serves for fixation of the overlap.

10. The wrist orthosis according to Claim 9,
**characterized in that**
to establish the overlap between the pairs of straps (14, 37), an engagement is designed in such a way that the straps (17, 20) of the supporting shell (10) engage in a strap guide (51, 52) formed on the straps (40, 42) of the complementary shell (34) or vice versa.

11. The wrist orthosis according to any one of claims 8 to 10,
**characterized in that**
the complementary shell (34) has two pairs of straps (37, 38) wherein the dorsal and/or volar straps (39, 41) of the distal pair of straps (37) are provided with a pressing device (54) which has a compressive force adjustment (64).

12. The wrist orthosis according to Claim 11,
**characterized in that**
the pressing device (54) is variable in its position relative to the strap (39, 41).

13. The wrist orthosis according to Claim 12,
**characterized in that**
the pressing device (54) is connected to the strap (39, 41) in a radially displaceable manner.

## Revendications

1. Orthèse de poignet (11), avec une coque de soutien (10) ouverte en direction radiale, pour réceptionner la zone distale de l'avant-bras et avec un appuie-main (29) volaire à poser contre la paume, ainsi qu'avec un dispositif de fermeture (31, 32, 33, 70) pour la liaison par complémentarité de force de l'orthèse de poignet avec la zone du poignet,
**caractérisée en ce que**
pour l'habillage de la coque de soutien (10), il est prévu un insert de soutien de forme stable, conçu pour épouser la zone de l'avant-bras, enveloppant la zone du poignet, qui en partant d'une partie destinée à l'avant-bras s'étend par delà la coque de soutien (10) avec une partie destinée au métacarpe (73) recouvrant la zone du métacarpe.

2. Orthèse de poignet selon la revendication 1,
**caractérisée en ce que**
la partie destinée au métacarpe (73) de l'insert de soutien est munie d'un dispositif (69) pour l'orientation fixante de l'insert de soutien sur le pouce.

3. Orthèse de poignet selon la revendication 1 ou 2,
**caractérisée en ce que**
l'insert de soutien est conçu dans une matière plastique élastique.

4. Orthèse de poignet selon la revendication 1 ou 2,
**caractérisée en ce que**
l'insert de soutien est conçu en un insert en coussin moulé (67) avec un remplissage en corps moulés composé d'une pluralité de corps moulés.

5. Orthèse de poignet selon l'une quelconque des revendications précédentes,
**caractérisée en ce que**
la coque de soutien (10) comporte côté ulnaire une zone de soutien (12) conçue en version continue, à partir de laquelle part au moins une patte (16, 17, 18, 19, 20, 21) en direction dorsale et en direction volaire.

6. Orthèse de poignet selon l'une quelconque des revendications précédentes,
**caractérisée en ce que**
la coque de soutien (10) est munie sur l'extrémité distale d'un bras cantilever (26) réglable dans sa disposition relative pour le placement de l'appuie-main volaire (29).

7. Orthèse de poignet selon la revendication 5,
**caractérisée en ce que**
le bras cantilever (26) est disposé de façon pivotante sur la coque de soutien (10), avec un axe de pivotement (74) s'étendant sensiblement de façon coaxiale par rapport à l'axe de la flexion dorsale.

8. Orthèse de poignet selon l'une quelconque des revendications précédentes,
**caractérisée en ce que**
pour le complément circulaire de la coque de soutien (10), il est prévu une coque complémentaire (34) ouverte côté ulnaire, qui côté radius comporte une zone de soutien (36) conçue sous forme continue, à partir de laquelle part au moins une patte (39, 40, 41, 42) en direction dorsale et en direction volaire et qui est susceptible d'être reliée par complémentarité de force avec la coque de soutien.

9. Orthèse de poignet selon la revendication 8,
**caractérisée en ce que**
pour la liaison par complémentarité de force de la coque complémentaire (34) avec la coque de soutien (10), il est prévu un recouvrement au moins partiel entre au moins un appariement de pattes (14) d'une patte dorsale et d'une patte volaire (17, 20) de la coque de soutien et un appariement de pattes (37) d'une patte dorsale et d'une patte volaire (40, 42) de la coque complémentaire, et **en ce que** le dispositif de fermeture (32) de la coque de soutien sert à fixer le recouvrement.

10. Orthèse de poignet selon la revendication 9,
**caractérisée en ce que**
pour la création du recouvrement entre les appariements de pattes (14, 37), il est conçu un engagement, de sorte que les pattes (17, 20) de la coque de soutien (10) s'engagent dans un guidage de patte (51, 52) conçu sur les pattes (40, 42) de la coque complémentaire (34) ou inversement.

11. Orthèse de poignet selon l'une quelconque des revendications 8 à 10,
**caractérisée en ce que**
la coque complémentaire (34) comporte deux appariements de pattes (37, 38), la patte dorsale et/ou la patte volaire (39, 41) de l'appariement de pattes distal (37) étant munie d'un dispositif presseur (54) qui comporte un réglage de la force de pression (64).

12. Orthèse de poignet selon la revendication 11,
**caractérisée en ce que**
le dispositif presseur (54) est variable dans sa position relative par rapport à la patte (39, 41).

13. Orthèse de poignet selon la revendication 12,
**caractérisée en ce que**
le dispositif presseur (54) est relié avec la patte (39, 41) en étant déplaçable en direction radiale.
